(19) Europäisches Patentamt
European Patent Office
Office européen des brevets

(11) Publication number: 0 396 788 A1

(12) EUROPEAN PATENT APPLICATION

(21) Application number: 89108264.6

(51) Int. Cl.5: C12M 1/40

(22) Date of filing: 08.05.89

(43) Date of publication of application: 14.11.90 Bulletin 90/46

(84) Designated Contracting States: AT CH DE ES FR GB GR IT LI NL SE

(71) Applicant: PPG HELLIGE B.V.
I.B.C.-Weg 1
NL-5683 PK Best(NL)

(72) Inventor: Kuypers, Martinus Henricus
Schoolstraat 53
NL-5561 AH Riethoven(NL)
Inventor: Steeghs, Gerardus Fransiscus Jozef
Nieuwendijk 54
NL-5662 AG Geldrop(NL)

(74) Representative: TER MEER - MÜLLER - STEINMEISTER & PARTNER
Mauerkircherstrasse 45
D-8000 München 80(DE)

# (54) Process and sensor for measuring the glucose content of glucosecontaining fluids.

(57) The process for measuring the glucose content of glucose-containing liquid media, in particular, for the in vivo measurement of the glucose content of blood, is based on the principle of a catalytic reaction in immobilized glucose oxidase in the area of the working electrode (5) of an electro-chemical sensor. Since oxygen is consumed in the reaction of glucose, but in the medium to be measured the oxygen partial pressure, which also influences the measuring signal, is too low, in particular, under anaerobic measurement conditions, the invention provides for the use of a pulsed polarization voltage between a higher level (A), at which excess oxygen is produced, and thus a decreasing oxygen partial pressure is compensated for, and a lower operating voltage level (B), at which only the catalytic glucose reaction in glucose oxidase takes place to form hydrogen superoxide, which is oxidized at the working electrode. The current flowing thereby is sensed in the phase of low operating voltage level (B) and evaluated as a measure of glucose concentration.

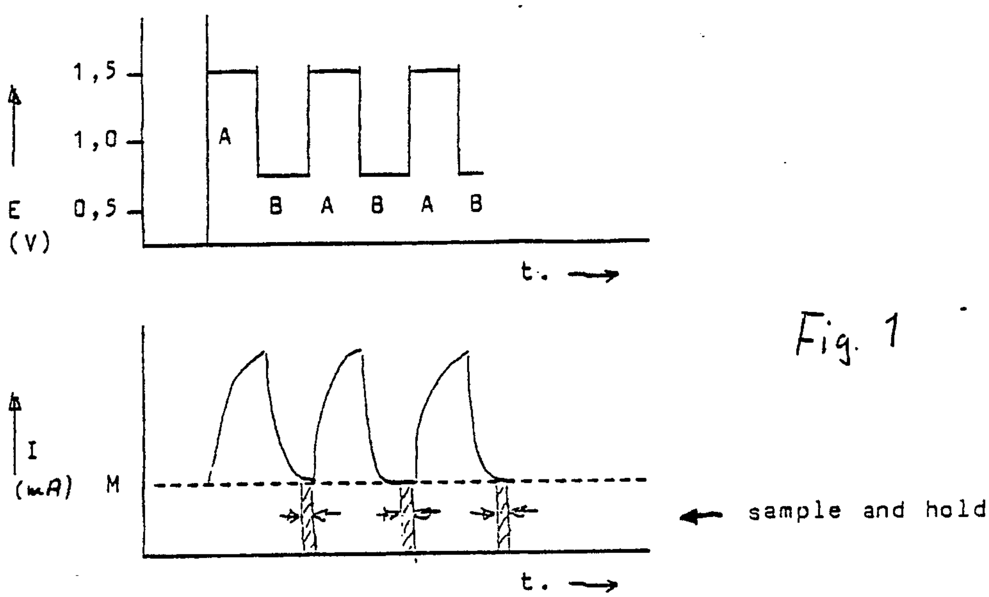


Fig. 1

EP 0 396 788 A1

## PROCESS AND SENSOR FOR MEASURING THE GLUCOSE CONTENT OF GLUCOSE-CONTAINING FLUIDS

The invention relates to a process and a sensor of very small dimensions for measuring the glucose content of glucose-containing liquid media, in particular for the in vivo measurement of the glucose content of blood under anaerobic conditions, based on the principle of a catalytic reaction in immobilized glucose oxidase.

A main problem with glucose sensors is that the signal values do not only depend on the glucose concentration but, inter alia, and in particular, also on the partial pressure of oxygen in the examined medium. For eliminating this $pO_2$ dependence, it has already been known to regenerate the consumption of oxygen electrolytically from the water contained in the enzyme layer surrounding the working electrode. To this end, a glucose diffusion controlled by using immobilizing media is applied with the use of a glucose electrode which is in reactive contact with an oxygen-generating galvanic oxygen electrode (cf. S.O. Enfors, Oxygen-stabilized enzyme electrode for D-glucose analysis in fermentation broths, Enzyme Microbiological Technology, January 1981, Vol. 3, pages 29 to 32). In doing so, the oxygen consumption of the enzyme electrode can be compensated for by the said combination of an electrolytic (enzyme) electrode with a $pO_2$ electrode as a reference for the oxygen stabilization of the enzyme electrode. The principle applied in this connection is as follows: When glucose diffuses into an immobilized enzyme layer in the anode area (working electrode) a local reduction of the dissolved oxygen ($pO_2$) is sensed by the built in oxygen electrode and a compensating electrolytic regeneration of oxygen is caused by a feedback-loop-type electronic monitoring circuit. The electrolytic current required therefor is used as the output signal in the glucose electrode and is proportional to the glucose concentration. In the case of this type of combination sensors, therefore the consumption of oxygen is determined as a measure of the glucose concentration in an examined sample.

The known Enfors combination electrode is however not suitable for in vivo measurements due to its size and the considerable technical expenditure alone. In this connection, it is also disadvantageous, above all, that a controlled (regenerated) stability of the surrounding oxygen partial pressure ($pO_2$) is a prerequisite for the signal current which is to be proportional to the glucose concentration. Therefore, the use under varying $PO_2$ as is to be considered in the case of in vivo measurements is out of the question.

A technically simpler method is the use of a non-oxygen-dependent glucose electrode with ferrocene being employed as electron acceptor in glucose oxidase (cf. Steven L. et al., Development of an on-line glucose sensor for fermentation monitoring, Biosensors, Vol. 3(1), 1987, pages 45 to 56). Ferrocene however suffers from the drawback that it is reported to be toxic by some authors, or at least its behavior in the human bloodstream and reactions on organs is unknown, and therefore does not come into consideration for numerous applications and for the time being, in particular in the field of medicine.

Generally, it is true for glucose sensors of the said type that the response time is determined by the diffusion time the glucose contained in the fluid to be measured (for instance, blood) requires to reach the working electrode. The diffusion/response time - especially when one thinks of in vivo sensors - must be lower than the physiological response time and must be in a certain relation to the rate of glucose metabolism change.

The reactions occurring at the working electrode of a glucose sensor are known. When the glucose which diffuses in from the glucose-containing medium reaches the working electrode area, the following reactions take place catalyzed by the immobilized glucose oxidase (G.O.D):

$$1/2\ O_2 + \text{glucose} + H_2O \xrightarrow{\text{G.O.D.}} \delta\text{-gluconolactone} + H_2O_2.$$

The hydrogen superoxide ($H_2O_2$) is oxidized at the working electrode:

$H_2O_2 \rightarrow 2H^+ + O_2 + 2e.$

The current measured is proportional to the glucose concentration. For the reasons stated above, the reactions which take place should not be limited by the glucose oxidase activity and the partial pressure of oxygen. This can be achieved, on the one hand, by immobilizing an ex cess glucose oxidase in the working electrode area. On the other hand, the oxygen partial pressure depends, of course, on the available amount of oxygen. When one thinks of an in vivo measurement with the use of very small sensors, in particular under anaerobic conditions, one must start out from the fact that the oxygen partial pressure to be expected is too low. At the moment, i.e. until better scientific results are available, the use of ferrocene is ruled out for

the reasons stated above because leakage or break of the electrochemical measuring cells can never be absolutely precluded.

Therefore, the object underlying the invention is to provide a process and a small-size glucose sensor for solving the above-shown problem of the compensating oxygen regeneration in the area of the layer surrounding a working electrode and containing glucose oxidase.

The solution according to the invention is described in Patent Claim 1 in respect of the measuring method according to the invention, with advantageous supplementations being defined in dependent patent claims.

A glucose sensor according to the invention for carrying out the process according to the invention is the subject-matter of Claim 8.

The process according to the invention provides that the sensor be operated with a pulsed alternating voltage switchable between a higher operating voltage level, preferably somewhat above 1.0 V, at which excess oxygen is released at the working electrode into the surrounding glucose oxidase by way of electrochemical splitting of water, and a low operating voltage level, preferably below 1.0 V, at which only the catalytic glucose reaction in glucose oxidase takes place to form hydrogen superoxide, which oxidizes at the working electrode, and the current flowing thereby is determined as the value sensed during the phase of low operating voltage level and is evaluated as a measure of glucose concentration.

In the case of the procedure according to the invention, the oxygen required for the biochemical reaction in the working electrode area is regenerated intermittently, followed by oxidation measurement for the $H_2O_2$ formed. In the phase of low voltage level, a signal can be sampled at the working electrode, this signal being proportional to the glucose concentration, and in the case of the proper control of the time phases and the proper selection of the higher voltage level, also independent of the oxygen partial pressure of the surrounding medium.

The time-correct sampling of the current values to be measured for the determination of the glucose concentration can be accomplished by means of a threshold-triggered sample-and-hold circuit. The evaluation itself is performed by means of a simple comparison programme. The glucose values are indicated by a digital display or a printer.

An important aspect for carrying out the invention successfully is the proper amount of excess immobilized glucose oxidase in the hydrogel layer surrounding the working electrode, the lowest layer directly upon the layer consisting of the reference electrode(s) and the working electrode on a passivation layer of a semiconductor substrate. The "doping" of the hydrogel layer, in particular, in the working electrode area of the sensor, is accomplished preferably by a photolithographic process as for example described in W.S., DeForest, Photoresist-Materials and Processes, McGraw-Hill Book Co., New York, pages 1 to 3 and with more detailed reference to the present application in EP 88 116 789.4. In numerous tests, on the one hand, by exposing immobilized G.O.D. to a glucose solution of defined concentration, the enzyme activity of this immobilized glu cose oxidase hydrogel layer, and on the other hand, the diffusion coefficient of glucose in the hydrogel, were measured first in a two-compartments cuvette and then in situ at the sensor. The immobilization of G.O.D. in hydrogel by a photolithographic process is likewise described in the above-referenced EPC application EP 88 116 789.4 as well as suitable hydrogels.

Glucose concentration is determined by spectrophotometry at a sample.

The chemical formula of G.O.D. is not known, but it can be described as a glycoprotein having a molecular weight of approximately 150,000 to 180,000. It contains 2 moles of flavin adenine dinucleotide (F.A.D.), per mole of enzyme and 11 to 16% carbohydrates.

When designing the sensor, which is intended preferably to be suitable also for in vivo (in tissue) measurements, the interest was focussed on a small-size oxygen sensor constructed on the basis of a semiconductor chip. A polarographic-amperometric sensor of this type which is preferably operated potentiostatically as a three-electrode sensor is described in European Patent Application EP 88 112 641.1 herewith referred to. A three-electrode sensor of this type is also of excellent suitability for the instant use as a glucose sensor. The results achieved to date are very promising.

In the case of the provided sensor constructed according to the planar semiconductor hybrid technique, the glucose pentrating through defined areas at the sensor is forced to reach the working electrode in lateral diffusion direction. The diffusion gradient thereby can be adjusted to the rate of conversion of glucose into glycogen in an immobilized enzyme layer on the surface of the working electrode.

For the provided in vivo use of the sensor, it is important, on the one hand, that thrombolytic reactions be prevented in the area of the measuring surface of the sensor, and on the other hand, a sufficiently rapid diffusion of the glucose from the measuring surface into the working electrode area surrounded by immobilized glucose oxidase in hydrogel be achieved, however in such a way that an approximately constant oxygen partial pressure in the hydrogel layer can be obtained. In this respect, it must be noted

additionally that the oxygen produced in the higher operating voltage phases (above 1.0 V) does not diffuse away quickly.

According to an advantageous embodiment of a glucose sensor of the invention, these problems and conditions shown above are achieved by a tri-layer foil upon the reference electrode and the working electrode applied according to the planar technique. As the lowest cover directly upon the working electrode and the reference electrode, respectively, a hydrogel layer is applied, which is mixed entirely, or at least in the working electrode area, with immobilized glucose oxidase, as has already been mentioned. In the case of pulsewise excitation according to the invention and thus pulsewise electro-chemical production of oxygen at the working electrode, the following reactions take place in the excitation phase at the higher operating voltage level (A):

At A: k ($H_2O \rightarrow 2H^+ + 1/2\ O_2 + 2e$).

In addition, the normal oxidizing reaction for hydrogen superoxide takes place:

$H_2O_2 \rightarrow 2H^+ + O_2 + 2e$.

In phase (B), i.e. in the period of switching over to a lower operating voltage level, only the oxidizing reaction for hydrogen superoxide takes place, namely in accordance with the foregoing:

$H_2O_2 \rightarrow 2H^+ + O_2 + 2e$.

Upon the hydrogel layer, a hydrophobic membrane is applied which is only slightly permeable for oxygen, but impermeable for glucose, and which covers the entire hydrogel layer. To prevent thrombolytic reactions and penetration of proteins, the hydrophobic membrane is covered by a further hydrogel membrane containing heparin immobilized in a manner known per se for preventing blood coagulation. Examples of such heparinised polymers, inclusive of literature, are described in detail in European Patent Application EP 88 116 789.4 already mentioned. Reference is made to this prior European patent application, in regard of the disclosure of such hydrogel membranes containing immobilized heparin.

In the reference electrode(s) area, the hydrophobic membrane beneath the uppermost membrane is provided with windows. The glucose (and oxygen) diffusion is then performed through the hydrogel membrane mixed with heparin via the windows in the hydrophobic membrane into the lowest hydrogel layer, and furtheron, in lateral direction to the glucose oxidase reaction in the working electrode area.

A detailed description of the invention and advantageous details are given hereinbelow by way of illustrative embodiments, reference being made to the drawing.

Fig. 1 shows two time-correlated signal diagrams with an operating voltage switchable between two levels (at the top) and a signal current response (at the bottom);

Fig. 2 shows the diagrammatic design of a glucose sensor with features according to the invention;

Fig. 3 shows an enlarged detail from Fig. 2 for illustrating the processes of glucose and oxygen diffusion into the working electrode area of the sensor;

Fig. 4 shows a modified embodiment of Fig. 3, in which the G.O.D. containing hydrogel is surrounded by flux-rate limiting hydrogel;

Fig. 5 shows a greatly enlarged top view on a glucose sensor with features according to the invention based on a semiconductor chip;

Fig. 6 shows an even more greatly enlarged top view on the planar layer structure of a glucose sensor of the type according to the invention;

Fig. 7 shows a block diagram of an excitation and measurement circuit; and

Fig. 8 shows another ambodiment for an excitation and monitoring circuit for monitoring the glucose content under controlled oxygen content.

First of all, the principle of the process according to the invention is illustrated by means of the two signal diagrams shown in Fig. 1.

As has already been mentioned, the current flowing over the working electrode during the oxidation of $H_2O_2$ is proportional to the glucose concentration. Even though the $H_2O_2 \rightarrow 2H + O_2 + 2e$ reaction is not directly influenced by the $pO_2$ as long as the amount of oxygen is in excess in respect to the enzymatic reaction, this proportionality applies, however, only when the oxygen partial pressure in the area of the hydrogel layer mixed with glucose oxidase and surrounding the working electrode is approximately constant. Since, in the tissue to be measured, the oxygen partial pressure to be expected is too low, a two-step measuring method is applied according to the invention, wherein the polarization voltage is switched back and forth between different values. In a higher operating voltage phase (somewhat above 1.0 V) denoted by A in Fig. 1, on the one hand, an oxidation reaction takes place for the $H_2O_2$ obtained in the reaction of glucose oxidase. On the other hand, however, excess oxygen is released into the hydrogel layer surrounding the working electrode. The measuring current over the working electrode increases, which current results from and is proportional to the generated oxygen amount and the simultaneous electrochemical oxidation of $H_2O_2$; see lower diagram in Fig. 1. The free oxygen released to the hydrogel layer

is partly reconsumed in the enzymatic reaction which takes place continuously and a part of this oxygen diffuses through the polymers to the physiological medium (blood), if $pO_2$ in that medium is low. The voltages during the time periods A are to be selected above 900 mV and preferably a few hundred mV above 1 V at which water decomposes. The upper limit of this voltage may be defined by the quantity of oxygen generated and too high voltage may damage the triple-layer polymer concept described below in conneotion with Figs. 4 to 6. Also, at voltage levels well above 1 V, the generated hydrogen may cause problems if a potentiostatic driving concept for the sensor is used with an outside metal case serving as a counterelectrode.

In the phase B of switching over to the lower polarization voltage level (below 900 mV or 1.0 V), only a glucose reaction (G.O.D. reaction) takes place, which results in δ-gluconolactone and $H_2O_2$ being formed, whereby the $H_2O_2$ is again oxidized at the working electrode. At this lower operating voltage level, the oxygen amount corresponding to the conversion reaction is consumed and the current which flows in this operation phase corresponds to the glucose concentration. As is shown in the lower diagram in Fig. 1, momentaneous values of the current amplitude which is proportional to the glucose concentration can be sampled by time-correct sensing.

As to the duty cycles for switching A-B-A ... tests show that the lower limit will be a few seconds but also larger periods in the range of one minute or more are possible depending on the physiological response time of glucose concentration changes that may take several minutes. The sampling periods marked by hatched time slots in the lower diagram of Fig. 1 should be selected such that the capacity influence in particular caused by building up and reducing of ionic double layers has faded out.

Tests show that measurement of glucose with the principle according to the invention is possible under anaerobic conditions if, for example, oxygen is generated at the working electrode by pulsing (time periods A) during 100 s at voltages between 1.0 to 1.5 V. A stabilizing current was observed due to the oxidation of $H_2O_2$ only after switching to an excitation of 0.7 V (time periods B). Under the test conditions, during which the working electrode was covered with a G.O.D. albumen layer only, a linear relation could be established between the current and the glucose concentration up to 80 mg/dl.

It should be emphasized that the glucose reaction will take place during phases A and B continuously. This reaction is not dependent on the electrochemical reaction, but on the excess amounts of $O_2$, G.O.D.-activity and the limiting diffusion flux. The schematic sequence of the reactions can also be written:

$E_{0x}$ + glucose $\rightarrow$ $E_{red}$ + gluconolactone

$E_{red} + O_2 \rightarrow E_{0x} \cdot H_2O_2 \rightarrow E_{0x} + H_2O_2$. The $H_2O_2$ will react electrochemically as mentioned above.

Fig. 2 represents a diagram of the structural design of a glucose sensor based on a $pO_2$ sensor as is described, inter alia, as a three-electrode sensor in the above-mentioned European Patent Application EP 88 112 641.1.

On the one hand, two reference electrode layers 7, preferably made of Ag/AgCl, as well as a working electrode 5, are applied onto a silicon semiconductor substrate passivated on its surface (cf. passivation layers 11 and 12 in Fig. 3). The electrode layer composed of the working electrode 5 and the reference electrode 7 is covered by a hydrogel layer 3 mixed entirely, but at least in the area of the working electrode 5, with immobilized glucose oxidase, as illustrated in Fig. 2 by reference numeral 4. The hydrogel layer 3, 4 is covered by a hydrophobic membrane 2 slightly permeable for oxygen, but impermeable for glucose. To avoid uncontrolled changes of the sensitivity of the sensor, it is already known from US-PS 4 492 622 to achieve the electrolytic activation of the hydrophilic polymer layer (hydrogel) 3, 4 via holes 6, for instance in the area of the reference electrodes. This principle is applied here, too.

Via these holes 6, also the diffusion of glucose into the hydrogel layer is controlled. The layer of the hydrophobic membrane 2 is covered by a further membrane 1 containing heparin immobilized and crosslinked by photo-active react ion to such an extent that macro-biological molecules like proteins cannot pass; forming thus a semipermeable membrane. The chemical composition of the hydrophobic membrane 2 may be photo-crosslinked cyclo-cis-polyisoprene.

Figs. 3 and 4 illustrate the course of the diffusion and of the reaction. From the surrounding medium, for instance blood, the glucose content of which is to be continuously determined, on the one hand, oxygen diffuses directly through the membrane layes 1 and 2 into the hydrogel layer 3, 4. On the other hand, glucose molecules diffuse through the hydrogel membrane layer 1 and the window(s) 6 into the hydrogel layer 4 mixed with glucose oxidase, in which hydrogel layer 4 the above-mentioned conversion reactions for glucose and $H_2O_2$, respectively, take place at the working electrode (W.E.). The glucose diffusion is, and must be, limited; however, inter alia, by selecting the size of the window(s) 6, the degree of crosslinking of the hydrogels 1 and 3 and sizes of these hydrogels in respect of the flow path covered by the continuous flowing glucose to the working electrode 5. In Fig. 4, the hydrogel layer 3 extends also above the hydrogel layer 4 around the working electrode 5 for limiting the flux rate. By these measures glucose diffusion can be

adjusted in such a way that under constant oxygen partial pressure during the measuring phase the response time is shorter than the glucose metabolism rate in the examined medium.

As is merely outlined in Figs. 3 and 4 by reference numeral 18, the chip body is surrounded by a metallized layer made, for instance, of aluminum, stainless steel or conductive plastic. This conductive outer coating forms a counterelectrode so that the sensor can be operated potentiostatically as a three-electrode sensor, as is described in European Patent Application EP 88 112 641.1 already mentioned.

The greatly enlarged top view in Fig. 4, the scale of enlargement of which can be seen from the inserted dimensions, illustrates an actual tested embodiment of a semiconductor sensor chip with features according to the invention. The centrally arranged working electrode 5 is connected via a connection pad 14. A temperature-sensing element 8, which is represented in Fig. 2 only by way of a symbol, is located (only by way of example) in the semiconductor substrate body 10 beneath a portion of the reference electrode 7 and is contacted via the connection pads 15, 16. The temperature-sensing element 8 is required for the reference-correct evaluation of the measuring signals, but is of no significance in connection with the invention. Semiconductor substrate 10 and the coating surrounding the outer surface of counterelectrode 18, respectively, is connected via a connection bond pad 13, which can be used to influence the potential level of the silicon substrate in respect to temperature, but is of no significance for the working of the glucose sensor itself. Last, the Ag/AgCl reference electrode 7 consisting of two portions is contacted via a connection piece 17. The covering of all three electrode portions by the hydrophobic membrane 2 is outlined in Fig. 4.

The enlarged representation of Fig. 6 illustrates further details of the electrode portions of the sensor chip. The temperature-sensing element 8 is divided in the known manner into two portions, this being, however, not of interest in connection with the invention since this is also known otherwise. The hydrogel layer 3 covers the electrodes 5, 7 in the configuration as can be seen from Fig. 6; in the area of the working electrode 5, the doping of the hydrogel layer with immobilized glucose oxidase is outlined. This applies accordingly to the two overlying layers of the hydrophobic membrane 2 and the heparin-containing semipermeable membrane layer 1.

The block diagram of Fig. 7 depicts the principal moduls or parts of a excitation and measuring circuit. The glucose sensor only shown as a block 19 is a three electrode sensor operated potentiostatically in a manner described in above-referenced European Patent Application EP 88 112 641.1. The negative input of operational amplifier 20 is connected to the output of a pulse generator supplying the phase A and phase B potentials in time sequence as described above in connection with Fig. 1. The varying measuring current at the working electrode leads to a voltage drop across a resistor 22 and this voltage value U is transferred to a sample-hold circuit 23 controlled in a manner as described in connection with the lower diagram of Fig. 1 to supply during the sampling time slots a voltage $U_c$ that is proportional to the glucose contents. This glucose proportional voltage $U_c$ can be digitized and processed in a manner known per se and can be transformed for display purposes.

A modification of the inventive concept disclosed above and in particular in connection with Figs. 1 to 4 and 7 is described in the following in connection with Fig. 8.

Recent scientific publications give some evidence that controll of the oxygen content may be valuable in respect to glucose concentration over 3.0 mM of glucose. It seems that the inactivation rate of the enzyme-hydrogen-peroxide complex increases with the oxygen concentration significantly (see Tse, Gough, Time-Dependent Inactivation of Immobilized Glucose Oxidase and Catalase, Biotechnology and Bioengineering, Vol. XXIX, pp. 705-713 (1987)). This leads to the assumption that it must be very valuable to switch over to an operation mode in which the oxygen can be monitored such that via a closed loop control the amount of the oxygen needed for the enzyme-glucose reaction can be controlled within narrow limits.

The block circuit diagram and the related time correlated operation diagrams show such a modified concept including an operation phase with oxygen monitoring. The operation phases A and B correspond in principle to those disclosed and described above in connection with Fig. 1. The third operation phase C serves for adjusting and monitoring the oxygen content to an optimum for the enzyme-glucose reaction. The various blocks of the circuit diagram of Fig. 8 are virtually the same as those described in connection with Fig. 7 with the exception that the pulse generator 21 generates and supplies to the negative input of operational amplifier 20 in a time sequence controlled manner a three level pulse signal in principle as shown in the upper diagram resulting in a current signal via the working electrode 5 shown in the middle diagram of Fig. 8. The lower diagram in Fig. 8 depicts the sample and hold values for the glucose concentration monitoring and the oxygen content monitoring, respectively, as output signals $U_1$ and $U_2$ of sample and hold circuit 23.

A further improvement of the inventive concept may be achieved by providing an extra electrode 24 close to the working electrode which is shown in Fig. 3 in phantom lines. At this extra electrode 24, the

oxygen can be generated continuously while at the working electrode 5 the glucose can be monitored continuously, or alternatively, the glucose and oxygen can be monitored sequentially.

Summarizing the above, the invention provides for a process and a small sized glucose sensor for glucose measurement with pulse generated oxygen at the working electrode under anaerobic conditions. Diminishing oxygen diffusion can be achieved by a second membrane which extends the linear relation range between measurement current and glucose concentration.

## Claims

1. A process for measuring the glucose content of glucose-containing liquid media, in particular, for the in vivo measurement of blood under anaerobic conditions, based on the principle of a catalytic reaction in immobilized glucose oxidase in the area of the working electrode (5) of an electrochemical sensor, **characterized in that** said sensor is operated with a pulsed alternating voltage switchable between a higher operating voltage level (A), at which excess oxygen is released at the working electrode (5) and into the surrounding glucose oxidase (4) by way of electrochemical splitting of water, and a lower operating voltage level (B), at which only the catalytic glucose reaction in glucose oxidase takes place to form hydrogen superoxide ($H_2O_2$), which oxidizes at said working electrode (5), and the current flowing thereby is determined as the value sensed in the phase of low operating voltage level (B) and is evaluated as a measure of glucose concentration.

2. The process as claimed in claim 1, **characterized in that** said sensor is operated with a three level time sequentially pulsed alternating voltage of which a third operating voltage level (C) is lower than said second lower voltage level (B), and in that the oxygen content released at said working electrode (5) into the surrounding glucose oxidase (4) is controlled and monitored during the time periods of said third operating voltage level (C).

3. The process as claimed in claim 1 or 2, **characterized in that** the glucose oxidase surrounding said working electrode (5) is immobilized by being deposited in a polymer or hydrogel layer (3, 4) sensitive to light and the degree of immobilization is adjusted by exposure to light, said exposure being controlled in respect of time and intensity.

4. The process as claimed in claim 3, **characterized by** the use of a glucose sensor designed according to the semiconductor planar thin-film technique with a tri-layer polymer coating covering said working electrode (5) and a reference electrode (7), wherein the first layer directly covering said electrodes (5,7) consists of a polymer or hydrogel into which the immobilized glucose oxidase is introduced at least in an area (4) surrounding said working electrode (5), the second layer is formed by a hydrophobic membrane (2), and the third, outer layer (1) is formed by a semipermeable hydrogen foil containing deposited therein immobilized heparin.

5. The process as claimed in claim 4, **characterized in that** for the controlled regulation of the diffusion of glucose from the liquid medium (blood) into said hydrogel layer (4) mixed with glucose oxidase at said working electrode (5) in the area of said reference electrode (7) the other controlled hydrogel layers (1, 3) are photo-crosslinked to a controlled extent, and a controlled size window (6) is provided in said hydrophobic membrane (2).

6. The process as claimed according to any one of the preceding claims, **characterized in that** said glucose sensor is designed as a three-electrode sensor and operated potentiostatically, wherein the third electrode used as a counterelectrode is formed by an outer conductive coating (18) surrounding at least partially the semiconductor base (10).

7. The process as claimed in any one of the preceding claims, **characterized in that** said higher operating voltage level (A) is above 1.0 V, preferably in the range from 1.0 to 2.0 V, and for said low operating voltage level (B), a value below 1.0 V, preferably in the range from 0.3 to 0.9 V, is chosen.

8. A glucose sensor for carrying out the process as claimed in at least one of the preceding claims, formed by a planar semiconductor substrate (10) in the form of a chip, which concurrently serves as a connection to said outer counterelectrode (i8) and as a shielding and which includes above a surface passivation (11, 12) a conductive reference electrode layer (7) and a working electrode (5), **characterized in that**

- said reference electrode (7) and said working electrode (5) are covered by a first polymer or hydrogel layer (3) containing deposited therein at least in the area of said working electrode (5) immobilized glucose oxidase,
- said first layer (3, 4) is covered by a hydrophobic membrane (2) only slightly permeable for oxygen, and that

- a semipermeable hydrogel membrane (1) permeable for glucose and oxygen, but impermeable for proteins, and mixed with immobilized heparin is applied above said hydrophobic membrane (2).

9. The glucose sensor as claimed in claim 8, **characterized in that** said hydrophobic membrane (2) is impermeable for glucose, but has at least one window (6) in the area of said reference electrode (7) for the electrolytic activation and the selective diffusion of glucose into said first hydrogel layer (3) and into said hydrogel layer (4) covering said working electrode (5) and mixed with glucose oxidase.

10. The glucose sensor as claimed in claim 9, **characterized in that** an auxiliary electrode (24) is provided in close proximity of said working electrode (5) for the purpose of continuous oxygen supply into said glucose oxidase containing layer around said working electrode (5).

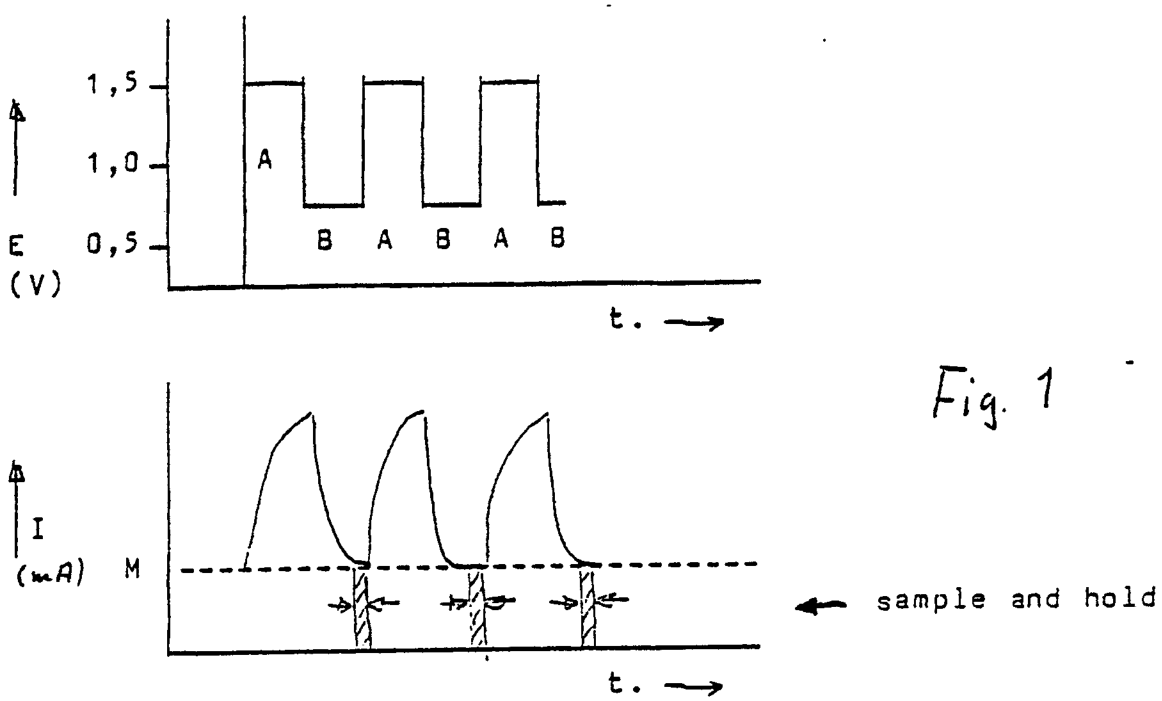


Fig. 1

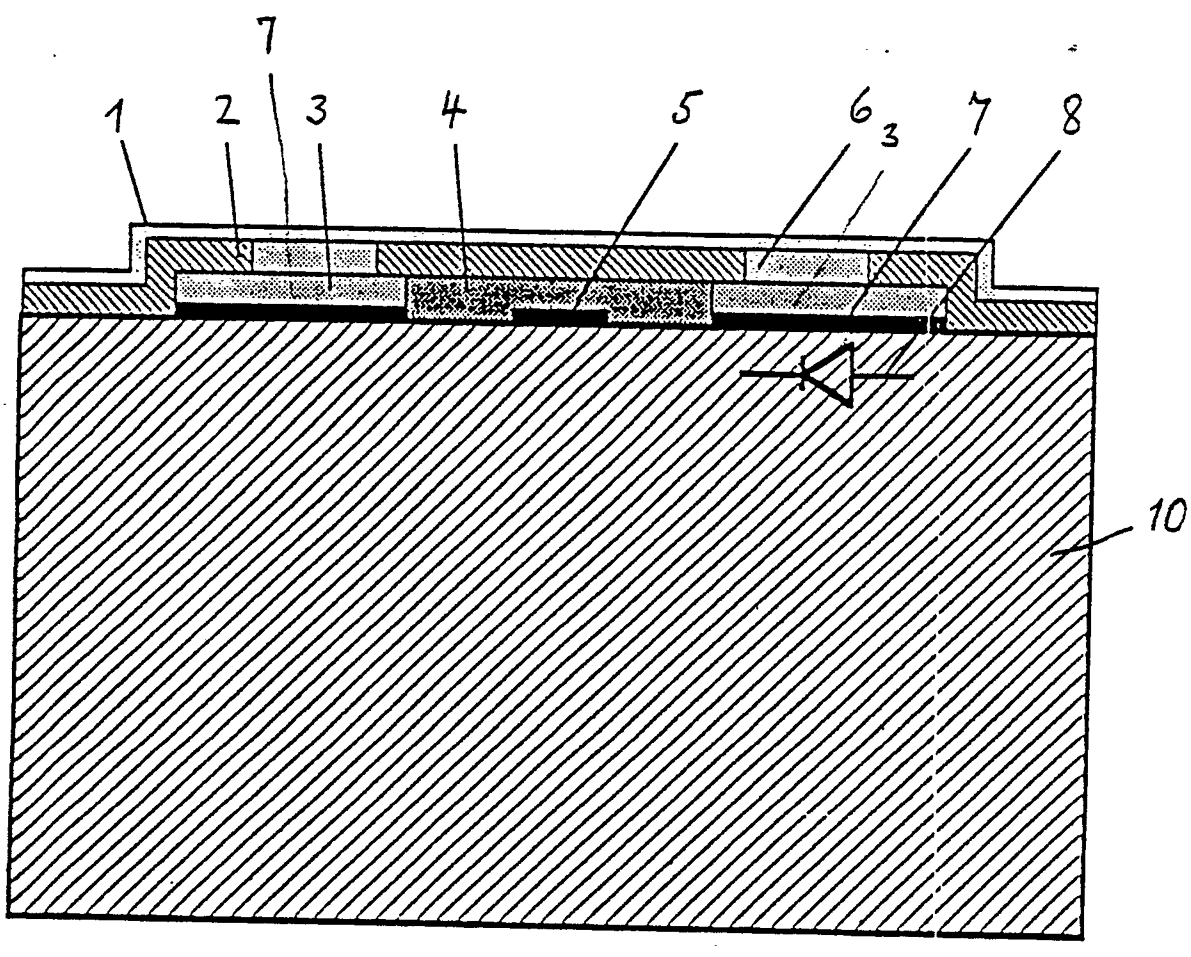


Fig. 2

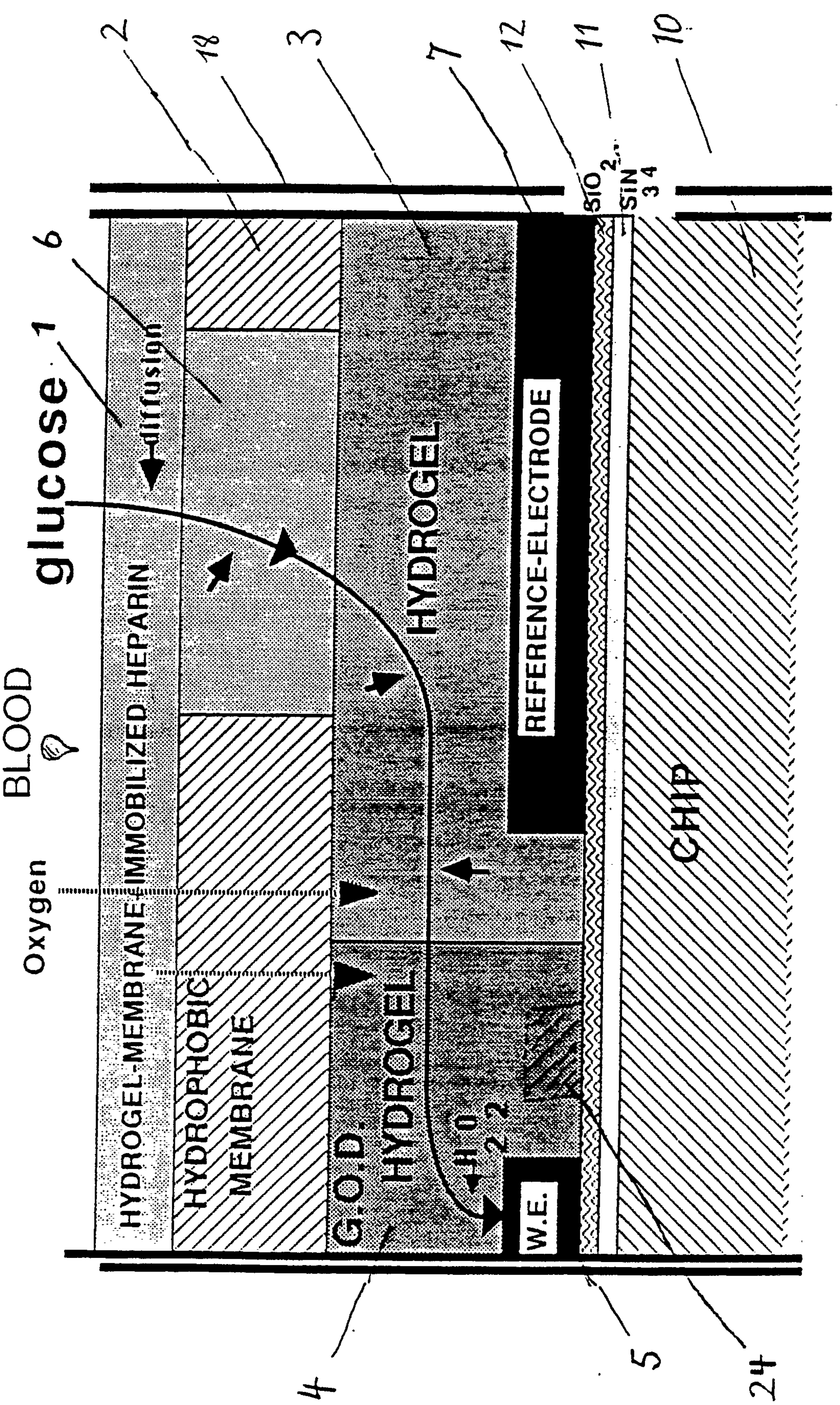
BLOOD
glucose
Oxygen
diffusion
HYDROGEL-MEMBRANE-IMMOBILIZED HEPARIN
HYDROPHOBIC MEMBRANE
HYDROGEL
G.O.D. HYDROGEL
$H_2O_2$
REFERENCE-ELECTRODE
W.E.
CHIP
$SIO_2$
$SIN_{34}$
1
2
3
4
5
6
7
10
11
12
18
24

Fig. 3

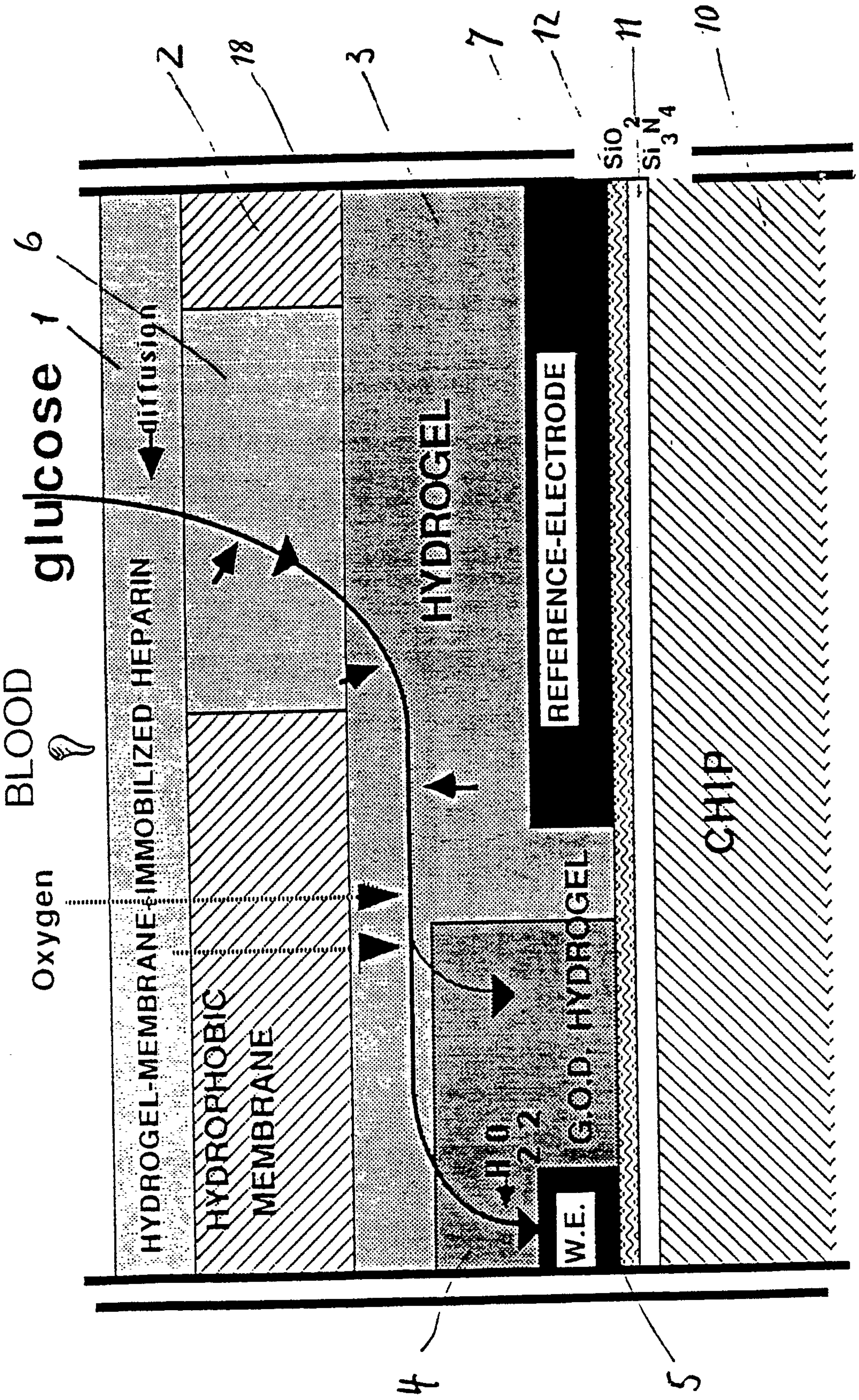
BLOOD
glucose
Oxygen
diffusion
HYDROGEL-MEMBRANE-IMMOBILIZED HEPARIN
HYDROPHOBIC MEMBRANE
HYDROGEL
REFERENCE-ELECTRODE
G.O.D. HYDROGEL
$H_2O_2$
W.E.
CHIP
$SiO_2$
$Si_3N_4$
1
2
3
4
5
6
7
10
11
12
18

Fig. 4

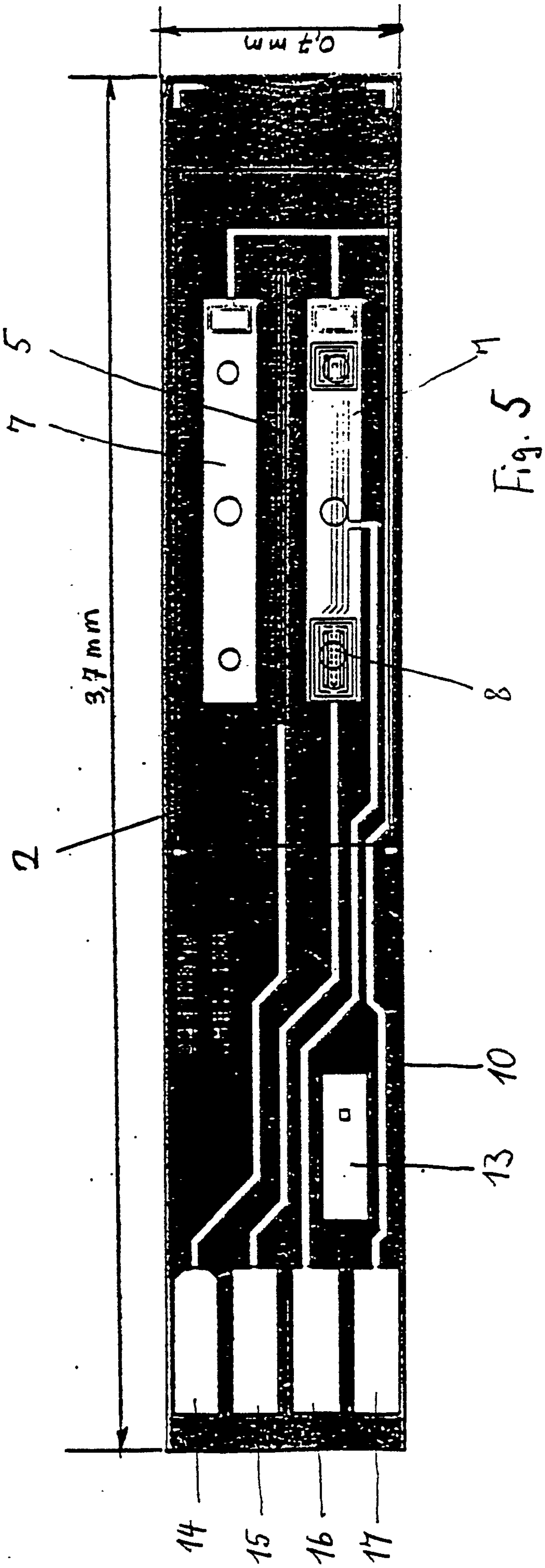


Fig. 5

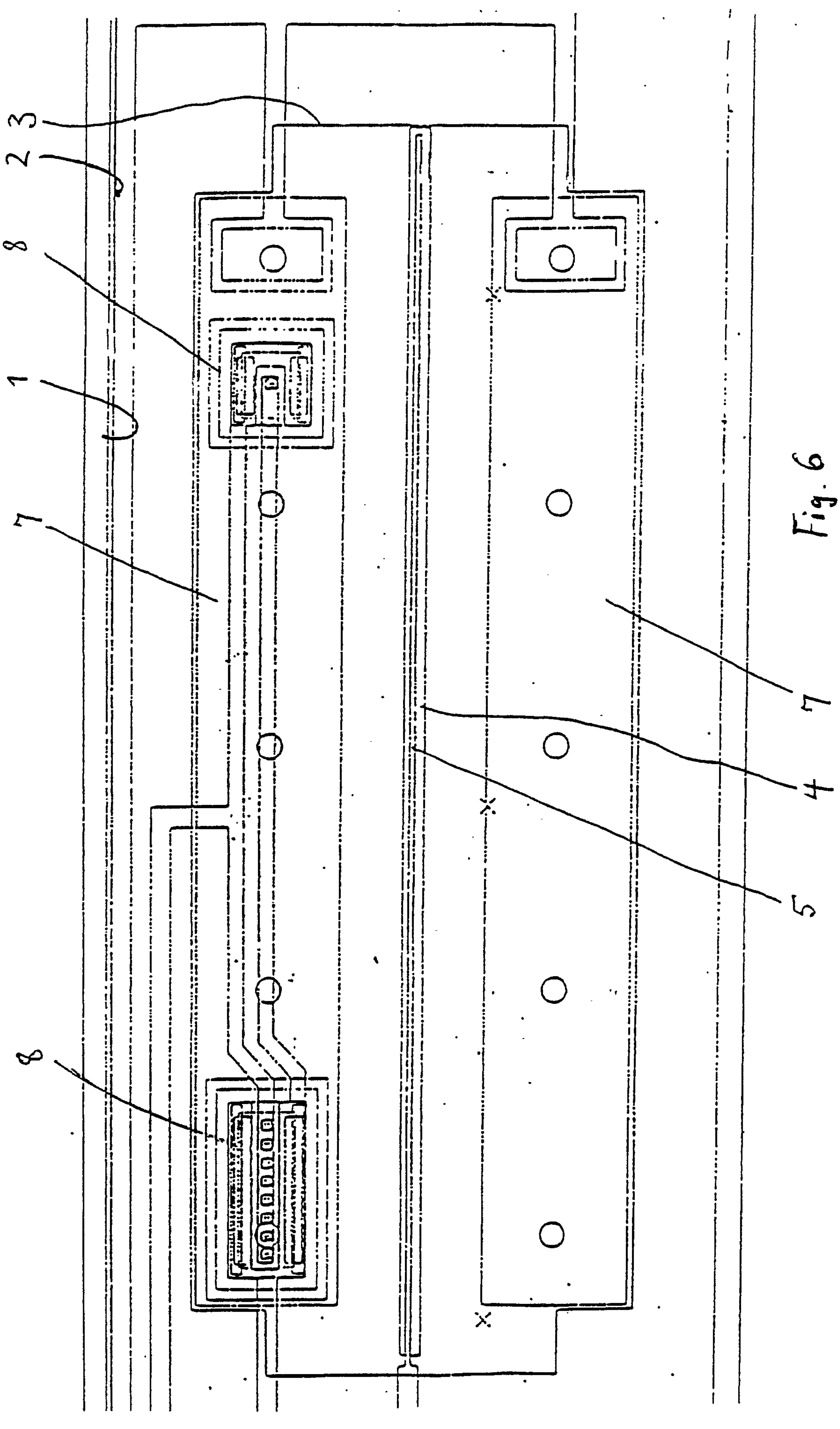


Fig. 6

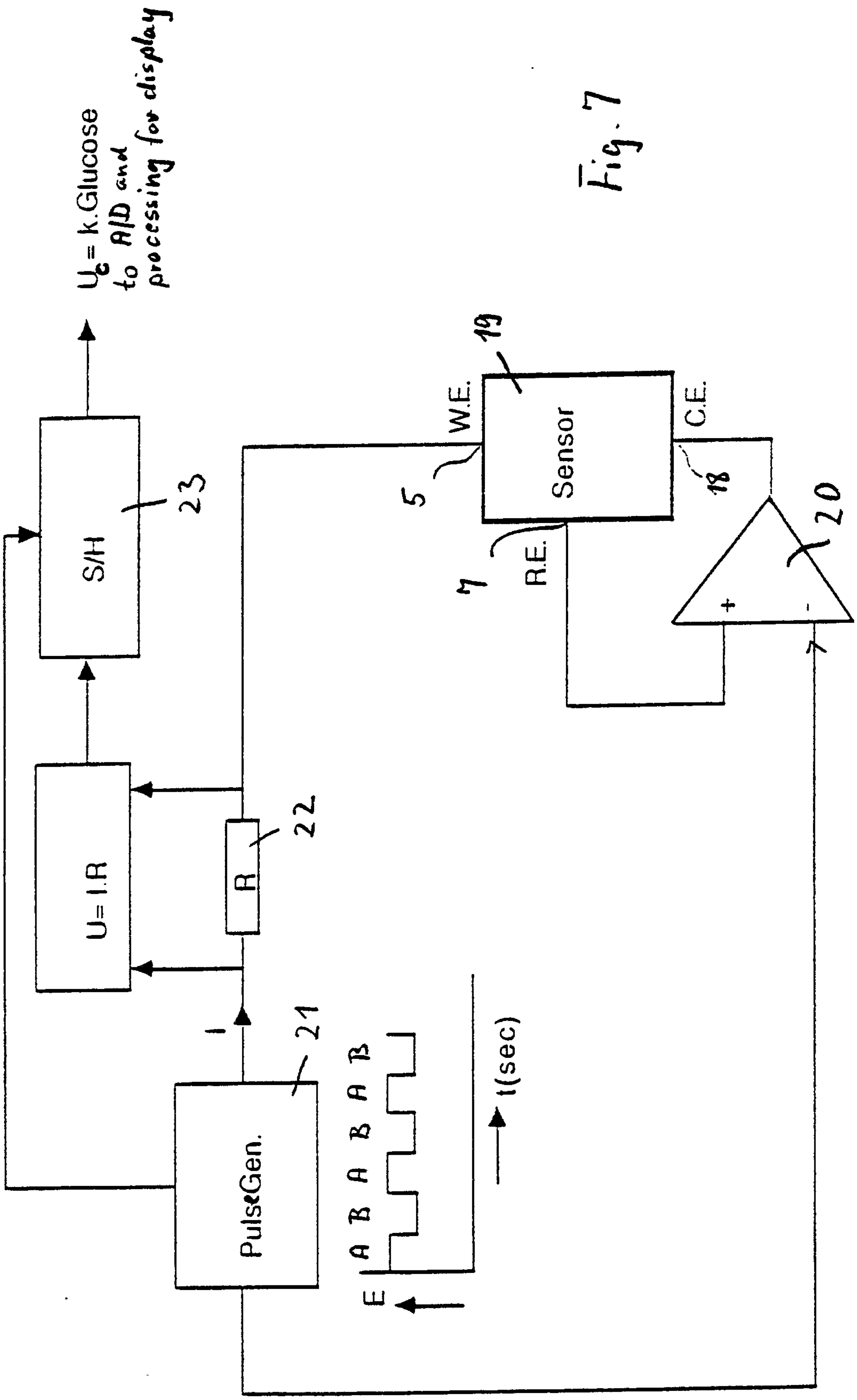
U_c = k.Glucose
to A/D and
processing for display
S/H
23
U= I.R
R
22
PulseGen.
21
I
A B A B A B A B
E
t(sec)
Sensor
19
W.E.
C.E.
R.E.
5
7
18
20
+
-

Fig. 7

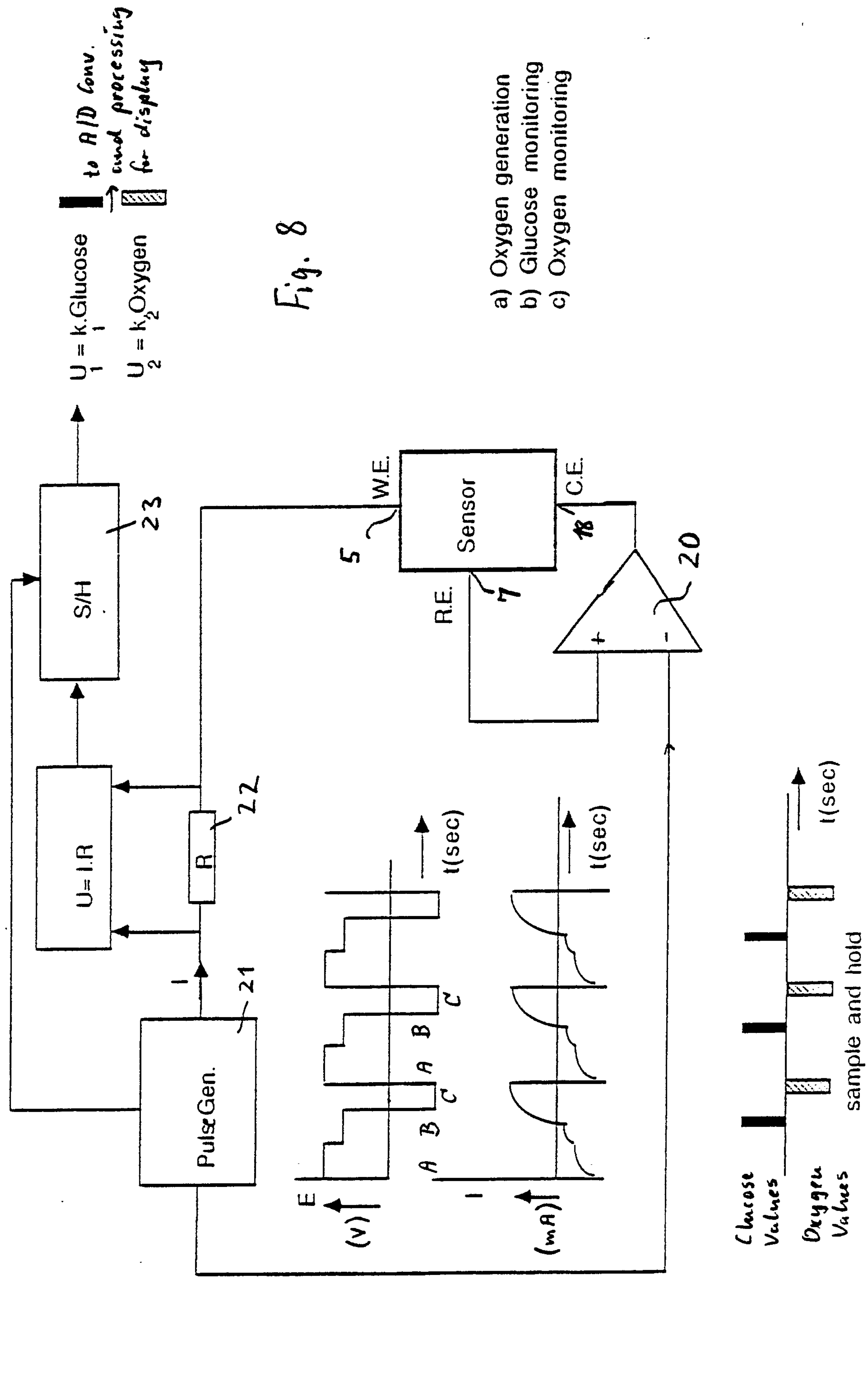
S/H
23
U = I.R
R
22
I
21
Pulse Gen.
$U_1 = k_1$.Glucose
$U_2 = k_2$Oxygen
to A/D Conv. and processing for displaying
W.E.
Sensor
C.E.
5
7
18
R.E.
20
+
-
E
(V)
A B C A B C
t(sec)
I
(mA)
t(sec)
Glucose Values
Oxygen Values
sample and hold
t(sec)
a) Oxygen generation
b) Glucose monitoring
c) Oxygen monitoring

Fig. 8

European Patent Office

# EUROPEAN SEARCH REPORT

Application Number

EP 89 10 8264

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 035 480 (ENFORS)<br>* Page 3, lines 19-32; claims 1-4 *<br>--- | 1-7 | C 12 M 1/40 |
| Y | EP-A-0 103 109 (SIEMENS AG)<br>* Claims 1-7; page 9, lines 28-33 *<br>--- | 1-7 | |
| Y | CHEMICAL ABSTRACTS, vol. 108, 1988, page 378, abstract no. 183280g, Columbus, Ohio, US; & JP-A-62 261 341 (FUJISAWA PHARMACEUTICAL CO., LTD. etc.) 13-11-1987<br>* Abstract *<br>--- | 3-6,8-10 | |
| Y | EP-A-0 284 518 (KARUBE)<br>* Claims 1,11,12,20,21,31,35 *<br>----- | 3-6,8-10 | |

TECHNICAL FIELDS SEARCHED (Int. Cl.5)

C 12 M
G 01 N

The present search report has been drawn up for all claims

| Place of search | Date of completion of the search | Examiner |
|---|---|---|
| THE HAGUE | 10-01-1990 | EPAILLARD P.J.H.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document